# EUROPEAN PATENT SPECIFICATION

(11) **EP 1 626 750 B1**
(45) Date of publication and mention of the grant of the patent: **21.07.2010**
(21) Application number: 04734638.2
(22) Date of filing: 24.05.2004
(51) Int. Cl.: A61L 27/34, A61L 28/00, A61L 29/08, A61L 31/10

(54) **BIOCOMPATIBLE POLYMER**
BIOKOMPATIBLES POLYMER
POLYMERE BIOCOMPATIBLE

(30) Priority: 23.05.2003 SE 0301546; 12.03.2004 US 552764 P
(43) Date of publication of application: 22.02.2006
(73) Proprietor: Gambro Lundia AB, 220 10 Lund (SE)
(72) Inventor: DEPPISCH, Reinhold, 72379 Hechingen (DE); DIETRICH, Ruth, 72379 Hechingen (DE); BECK, Werner, 72108 Rottenburg (DE); SCHNELL, Andrea, 72406 Bisingen-Thanheim (DE); WITTNER, Bernd, 72379 Hechingen (DE)
(74) Representative: Henriksson, Dan Ragnar Mikael
(86) International application number: PCT/SE2004/000804
(87) International publication number: WO 2004/103425

(56) References cited:
- EP-A2- 0 470 443
- WO-A1-00/21585
- WO-A1-00/64506
- WO-A1-02/13701
- WO-A1-03/000303
- WO-A2-03/047636
- US-A1- 2002 068 093
- US-B1- 6 267 782

## Description

### Technical field of the invention

The present invention relates to a biocompatible polymer composition for an article having a surface intended to contact blood, tissue, skin, epithelial layers, wounds, cells in culture fluids, body fluids, dialysis fluids and/or therapeutic fluids for removal or infusion.

The invention also relates to a method for the preparation thereof, an article comprising the biocompatible polymer composition and a use thereof.

### Background art

Many of the medical devices used in contact with blood, tissue, skin, epithelial layers, wounds, cells in culture fluids, body fluids, dialysis fluids and/or therapeutic fluids for removal or infusionare made of materials which are not biocompatible. Thus in many systems the materials are creating untoward reactions in the context of application in the respective biological system. Different types of application are e.g. transcutanous, in the peritoneal cavity, for access to the vascular system or in lines in which dialysis fluids are prepared.

Lack of biocompatibility may lead to blood clotting as well as inflammation and tissue activation and in addition, microbial infection can establish on the surface of devices. Colonization of bacteria and formation of biofilms on surfaces is a basic medical problem. Devices intended for long term contact, e. g. such as implanted stents, body fluid drainage systems or indwelling catheters can serve as a surface for host cell adhesion, permitting host cells to become activated, proliferate or to alter the normal physiological function and to restrict the function or intended use of a device. The formation of biofilms or bacteria colonisation on medical device surfaces creates a chronic inflammatory situation, which finally initiates failure of the device, and severe medical interventions or even life threatening situations.

The importance of antimicrobial activity and prevention of clot formation, e. g. in a catheter, has been disclosed in a paper by Wang et al, "Staphylococcus Epidermis Adhesion to Hydrophobic Biomedical Polymer is Medicated by Platelets", J. of Infectious Diseases, 1993, 167:329-36, where a strong relation is described between platelets deposition and promotion of bacterial growth.

GB-1 041 058 discloses a composition and a method for protecting materials against attack by fungi or bacteria, wherein a bismuth compound is applied to a surface, e.g. by spraying or tipping, or is incorporated into the material which is to be protected during fabrication thereof. The bismuth compound is used in applications with textiles, paintings, and disinfectant or to protect plants against attack by fungi and other microorganisms.

In US-A-5 928 671 is disclosed a series of bismuth salts having bactericidal and bacteriostatic activity for pharmacological use, antiseptic, antimicrobial and antibacterial agents for preventing infection and for disinfecting and cleaning surfaces, preservative and for killing biofilm organism and preventing the formation of biofilm. The composition is also used for treating bacterial infections of the gastro-intestinal tract.

A series of bismuth complexes, e. g. bismuth-propanedithiol or bismuth-pyrridione having antimicrobial and biofilm inibition properties, have been described by Domenico et al, " The potential of bismuth-thiols for treatment and prevention of infection", Infect. Med., 17(2):123-127, 2000. Said complexes are proposed to be used for coating of, e. g. indwelling catheters. Furthermore, Domenico et al have discussed the "Activities of bismuth thiols against Staphylococci and Staphylococcal biofilms", Antimicrobial Agents and Cemotherapy, May 2001, p.1417-1421.

WO 00/21585 discloses polycaprolactone, PDMS, as part of a polymeric film by the addition of a further component exerting antimicrobial activity and keeping the high biocompatibility profile of the coating (no cytotoxicity, improved thrombogenicity and reduced promotion of bacterial growth).

US-A-6,267,782 relates to a mixture of a metal composition and a biocompatible material in a solution for the preparation of a medical article comprising antimicrobial metal. The biocompatible material may comprise a biological polymer and the metal may be a bismuth composition. However, the metal composition is deposited on the surface of the article, resulting in release of bismuth from the article.

Prevention of blood access derived infections, e. g. in catheters is of great importance in public health perspectives, i. e. increasing resistance of bacteria against antibiotic strategies and with respect to costs related to subsequent medical treatment after bloodstream infections and septic complications. For example, intravascular catheter related bloodstream infections are an important cause of illness and excessive medical costs. Many catheter related bloodstream infections occur in intensive care units at the price of many deaths and high cost.

Therefore a lot of strategies have been developed to prevent these complications. As described by Donlan et al, "Biofilms and Device-associated Infections, Emerging Infectious Diseases, 89, Vol. 7, No. 2, March-April, 2001, most of these strategies to impregnate polymeric materials, e g by silver or other additives or even antibiotics, result in an ineffective control of bacteria growth and biofilm formation.

It is described by Mermel et al, "New Technologies to Prevent Intravascular Catheter Related Bloodstream Infections", Emerging Infectious Diseases, Vol. 7, No. 2, March-April, 2001, that technological interventions by impregnating catheter materials with different kinds of bacterial agents is not effective. In vitro studies have suggested the potential for bacterial resistance against the antimicrobial agents used to impregnate these catheters as their clinical use becomes more widespread. In addition to these very often non-technological inventions such as nurse training and use of sterile environment by sterile masks, sterile clothes, etc helps to reduce catheter related infections.

However, there is no technical solution available at the moment preventing, at the catheter site, the formation of biofilms by bacterial adhesion and proliferation. From pharmaceutical textbook knowledge, many bismuth compounds are used in medical and/or pharmaceutical practice e g bismuth carbonate, bismuth -nitrate, bismuth -citrate, bismuth-salicylate. Related drug formulations are known as Angass-S-Ulcowics, Bismoflk-V, Jadrox-600, Ulcolind, etc. Bismuth salts and thiols are active against a broad spectrum of bacteria. The inhibitory concentration is in the range of 3 to 300 µmol bismuth-3+. Most of the known bacteria strains are susceptible to bismuth compounds and it is of importance to note that they are most effective against Staphylococcus Aureus including methicillin resistant Staph. aureus (MRSA)(Dominico et al).

The main problem is that bismuth compounds, especially bismuth thiols are potentially toxic. The mechanism how bismuth is working to prevent bacterial proliferation is not completely clear. It was recently shown that Bis-BAL could enhance phagocytotic uptake of bacteria by neutrophils. Furthermore it has been shown that this compound could significantly enhance complement binding to cells and by this accelerate opsonisation and phagocytosis. However, this mechanism cannot be applied to prevent bacterial growth in aqueous solution. Therefore, a specific effect of bismuth must act on bacteria proliferation. It has been proposed that bismuth inactivates respiratory enzymes in the cytoplasma and by this leads to inhibition of capsular polysaccharide expression in bacteria. These polysaccharides are necessary to form a gel like autolayer surrounding the bacteria and preventing the action of antibiotic. Furthermore, it is advantageous that bismuth does not destroy the bacterial cell membrane and by this prevents the release of endotoxins which are known as an important stimulator of the immune system, especially in dialysis patients or patients depending on extracorporeal treatment during intensive care therapies.

Based on these findings, there is a clear medical need to design materials or surfaces in medical devices, especially in catheters, access devices or port systems, which prevent bacterial growth and subsequent biofilm formation and prevent bioincompatible reactions, especially formation of clots and fibrin or platelets deposits. To produce medical devices resistant to infections, a potent antimicrobial efficiency combined with an excellent biocompatibility over time is needed.

### Summary of the invention

The object of the present invention is to provide a biocompatible polymer composition for an article having a surface intended to contact blood, tissue, skin, epithelial layers, wounds, cells in culture fluids, body fluids, dialysis fluids and/or therapeutic fluids for removal or infusion, wherein the above mentioned drawbacks and problems have been eliminated or at least alleviated.

Thus, it is an object of the present invention to provide a biocompatible polymer composition capable of preventing bacterial adhesion and proliferation including biofilm formation.

This object has been achieved by the biocompatible polymer composition for an article having a surface intended to contact blood, tissue, skin, epithelial layers, wounds, cells in culture fluids, body fluids, dialysis fluids and/or therapeutic fluids for removal or infusion, characterized in that the polymer composition comprises a bismuth complex is incorporated in an amount corresponding to 0.002 to 0.08 weight% bismuth of the polymer composition.

Another object of the present invention is to provide a method for the preparation of the biocompatible polymer composition.

This object has been achieved by a method for the preparation of a biocompatible copolymer composition, characterized in that a bismuth complex is incorporated into the polymer composition in an amount corresponding to 0.002 to 0.08 weight% bismuth of the polymer composition.

Yet another object according to the invention is to provide an article having a surface intended to contact blood, tissue, skin, epithelial layers, wounds, cells in culture fluids, body fluids, dialysis fluids and/or therapeutic fluids for removal or infusion.

This object has been achieved by an article, characterized in that said article has a film of a polymer composition comprising a bismuth complex in an amount corresponding to 0.002 to 0.08 weight% bismuth of the polymer composition, covering said surface.

A further object of the invention is to provide a use of a biocompatible polymer composition.

This object has been achieved by the use of a biocompatible polymer composition comprising a bismuth complex incorporated into the polymer composition, for a medical device intended to contact blood, tissue, skin, epithelial layers, wounds, cells in culture fluids, body fluids, dialysis fluids and/or therapeutic fluids for removal or infusion in order to enhance biocompatibility and prevent bacterial growth. The biocompatible polymer of the invention may e.g. be used on surfaces in contact with blood, tissue, skin, epithelial layers, wounds, cells in culture fluids, body fluids, dialysis fluids and/or therapeutic fluids for removal or infusion.

The present invention shows a possibility to create antimicrobial biocompatible polymer compositions for any medical devices by means of bismuth components in polymer systems.

Another advantage of the invention is that the addition of Bi influences the polymer film composition and orientation of physicochemical domains in the surface, e.g. by catalysing the polymer forming reaction and thus allowing different functions.

Other distinguishing features and advantages of the invention will appear from the following specification and the appended claims.

A specific advantage derives from the process of coating/reactive polymer film making on a medical device containing an active compound in the thin crosslinked polymer layer. The ratio of base polymer substratum against the thickness of the polymeric film coating defines important properties of the medical article related to general function, biocompatibility and antimicrobial activity.

### Short description of the drawings

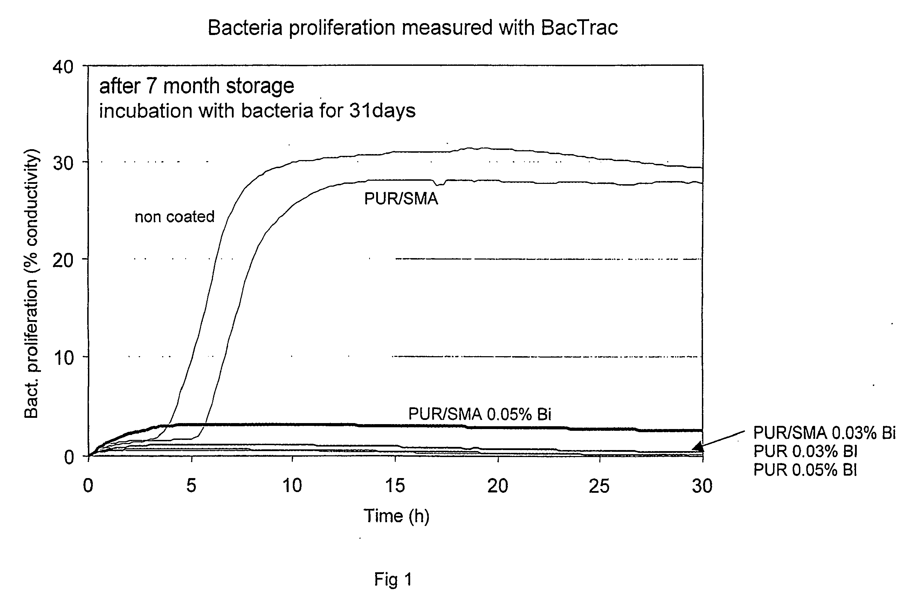

The invention will be described in greater detail below by means of the accompanying drawings, wherein
Fig 1 is a graph of bacteria proliferation for an article coated by PUR/SMA with 0.03% Bi incorporated therein versus a non-coated article.

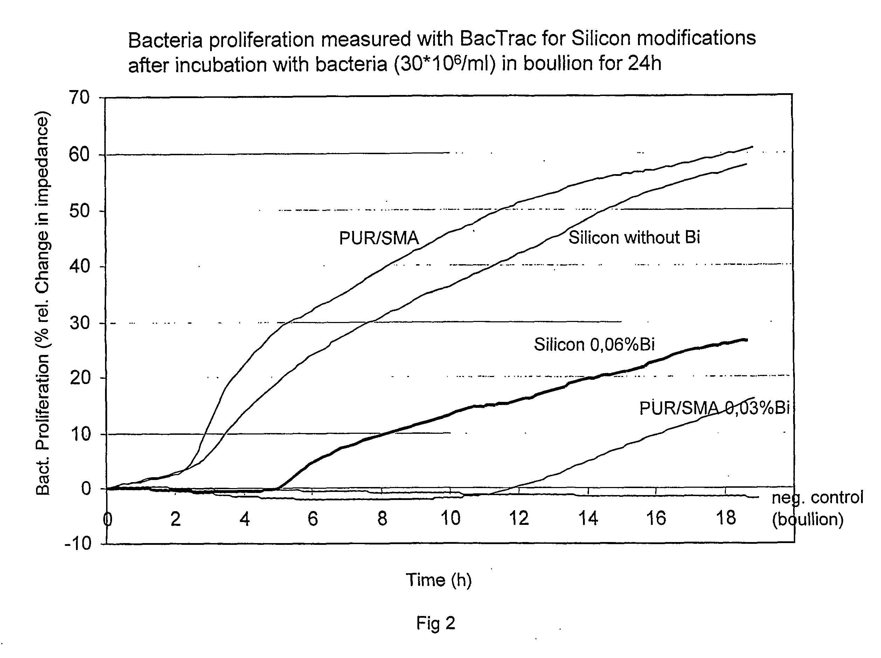

Fig 2 is a graph of bacteria proliferation for a silicon article with 0.06% Bi incorporated therein versus a non-coated silicon article, an article coated by PUR/SMA with 0.03% Bi incorporated therein and a non-coated article.

### Detailed description of preferred embodiments

The present invention includes a polymer composition, which can be applied as a film over a surface of an article to form a continuous surface which is more biocompatible and has a smoother surface morphology than an untreated article. Such polymer film can be formed by providing a hydrophobic polymer block, such as polydimethylsiloxane (PDMS) with two or more functional -OH end groups and reacting the -OH ends with a conventional monomer or prepolymer of a film-forming polymer capable of reacting with -OH groups. Such reactions are exemplified, using as reactive PDMS a triblock copolymer of the polylactone-polysiloxane-polylactone (PL-PDMS-PL) type or silicone polyesters. The -OH groups of the polylactone blocks can react with any of a variety of isocyanates in a suitable solvent to form a polymer having PDMS incorporated with its structure. The film can be applied to the surface of an article by any convenient means of coating the article with the reaction mixture in solvent, and allowing the solvent to evaporate.

The copolymer film can be prepared by reaction of a hydrophobic polymer block, for example a PDMS-containing block copolymer having reactive -OH groups, with a monomer or prepolymer of a film-forming polymer, for example, an isocyanate-polyol mixture. Suitable hydrophobic polymer blocks include various siloxane polymers, siloxane oligomers, fluoropolymers, polyethyleneglycols, polyethyleneglycol polydimethylsiloxane copolymers, silicone polyesters, polylactone-polysiloxane-polylactone triblock copolymers, polyamides, polysulfones, polyarylethersulfone, polycarbonates, polyolefins including cycloolefine-copolymers and the like. Basically all kinds of block copolymers can be applied for coating films according to the described invention. Reactive end groups on the hydrophobic polymer block react with monomer or prepolymer units of the film forming polymer. Alternatively, coupling agents can be used to react with the hydrophobic block and then with monomer or prepolymer units of the film-forming polymer.

Examples of film-forming polymers include polyurethanes, polyolefins, elastomers, polyethyleneglycols, polycarbonates, polyethersulphones, polyvinyl pyrrolidones, polyvinyl chlorides, polyamides, polysulfones, polyarylethersulfones, cellulosic polymers, cycloolefincopolymers, siloxane polymers and siloxane oligomers, and the like. Preferred are polyurethanes (PUR), which can be formed by reaction of isocyanate with a polyol. PL-PDMS-PL has -OH groups, which allow it to be incorporated internally into a polyurethane by reaction with free isocyanate groups. In order to create more or multiple dimensional crosslinking of the PUR system PDMS-polymers or copolymers with more than two OH groups can be applied. One example of oligomers of this type is disclosed in EP 0 294 525, which is hereby included by reference.

In a preferred embodiment of the present invention the polymer composition contains polyisocyanate-prepolymer with a NCO-content of 1-60% which is reacted with a OH-group of a polymer containing hydrophobic domains such as triblock-copolymer of polycaprolactone-polydimethylsiloxane-polycaprolactone of molecular weight in the range 100-100,000.

Triblock copolymers having a polydimethyl siloxane (PDMS) block flanked by polylactone (PL) blocks have been described by Lovinger, J. et al (1993), J. Polymer Sci. Part B. (Polymer Physics) 31:115-123. Such triblock copolymers have been incorporated into bulk formulations, and also applied as surface coatings, to reduce thromogenicity, as described in US-A-5 702 823. PL-PDMS-PL triblock copolymers are commercially available, for example from Thoratec Laboratories, Berkley, Calif., which provides a series of such polymers designated SMA in which the siloxane is dimethyl siloxane and the lactone is caprolactone, and from Th. Goldsmith AG, Essen, Germany, under the name TEGOMER (trademark, Goldsmith AG). The nominal molecular weights (number average) of the polysiloxane blocks suitable for use herein range from about 1000-5000, while the nominal molecular weights of the caprolactone blocks range from about 1000 to about 10,000. Tsai, C-C. et al (1994) ASAIO Journal 40:M619-M824, reported comparative studies with PL-PDMS-PL blended into polyvinyl chloride and other base polymers or applied as a coating thereon.

In this reactive mixture bismuth containing salts, thioles or other bismuth-complexes are added to form a mechanically stable film which can react in presence of humid air to accelerate the polymer forming reaction. The concentration of the bismuth complexes should be in the range corresponding to 0.002 to 0.08 weight% bismuth of the polymer composition.

The bismuth complexes may be accumulated in the outer layer of the film. Without being bound to any theory it is suggested that the bismuth complexes could migrate in a direction away from the film surface in order to accomplish equalization of the concentration of bismuth complexes throughout the film. In order to prevent this, in a preferred embodiment of the invention, nano-particles including a bismuth complex are further added to the polymer composition as a complement in order to achieve a slow-release of bismuth complex. By the presence of nano-particles containing bismuth complex it is possible to delay depletion of bismuth complex from the polymer film surface. The nano-particles may be prepared from polylactic acid. By controlling the degree of polymerisation of the polylactic acid it is possible to control the rate of the release of bismuth from the nano-particles.

In another preferred embodiment of the invention a catheter is provided with a first film with nano-particles having slow-release of bismuth complex incorporated therein. Subsequently a second film is provided with free bismuth complexes. The slow release of bismuth complexes from the first film then prevents the migration of the free bismuth complexes in a direction away from the film surface, inwards into the polymer film.

The invention also provides a method of coating an article with a polymer film, by combining a film-forming polymer composition and a bismuth complex with a hydrophobic polymer block having end groups reactive with the film-forming polymer component in the presence of a solvent such that all components are dissolved in the solvent. Subsequently, the components dissolved in the solvent are incubated under conditions to allow the components to react with one another in solution. Finally, a film is formed by spreading the solution over a surface to be coated under conditions that allow the solvent to evaporate.

More than one film may be formed on the surface to be coated. It is also possible for the different film layers to have different thickness. Furthermore, the concentration of bismuth in the different layers may also vary. In this way it is possible to achieve a desired distribution profile for the bismuth complex. The thickness of the films may be in the range from about 1-100 µm, preferably in the range from about 5-50 µm.

The invention is carried out by using a commercially available PL-PDMS-PL, a triblock copolymer of polycaprolactone-polydimethylsiloxane-polycaprolactone such as TEGOMER H-Si 6440 (trademark, Th. Goldsmith A. G., Essen, Germany,) and adding a bismuth containing salt, thiol or other bismuth complex thereto.

In an alternative embodiment the invention is carried out by using siloxane polymers and/or siloxane oligomers with the addition of a bismuth complex.

Examples of suitable bismuth complex are chosen from the group comprising ammonium bismuth citrate, bismuth(III)oxide, bismuth(III)gallate hydrate, bismuth citrate, bismuth(III)oxychloride, bismuth(III)tetramethylheptanedionate, bismuth(III) hexafluoracetonate, bismuth(III)subsalicylate, triphenylbismuth, bismuth(III) ciprofloxacin, bismuth(III)chloride, triphenylbismuth dichloride, triphenylbismuth carbonate, triphenylbismuth dihydroxide, triphenylbismuth dinitrate, triphenylbismuth disalicylate, triphenylbismuthine, triphenylbismuth bis(2-chloroacetate), triphenylbismuth bis(4-aminobenzoate), bis(acetato-O)triphenylbismuth, dibromotriphenylbismuth, and difluorotriphenylbismuth. Bismuth thiols such as bismuth propanedithiol, bismuth pyrithione and bismuth dimercaptotoluene, etc, may also be used. The concentration of the bismuth complexes should be in the range corresponding to 0.002-0.08 weight% bismuth. In a preferred embodiment of the invention the bismuth complex is triphenylbismuth or triphenylbismuth dichloride.

Subsequently, the Bi-containing PL-PDMS-PL triblock copolymer is reacted with a polyurethane (PUR) prepolymer (DESDOMUR E23, Trademark, Bayer Co.), wherein PL-PDMS-PL blocks react as bifunctional units that become incorporated internally in the PUR polymer chain.

The -NCO content should be within the range of 1-60 weight%, more preferably 5-20 weight% and most preferably 7-16 weight%.

The formulation is used to prepare, e. g. a film or a coating or a surface which film or coating is chemically crosslinked, mechanically stable, elastic, non-toxic, exerts inhibition of bacteria growth in comparison with films or coatings without bismuth complexes and reduced thrombogenicity in comparison with uncoated surfaces.

Alternatively a Bi-complex may be added as an additive in injection moulded parts. Other technical processes like casting or extrusion of films, plates or multilayer tubular materials are suitable to create the described polymer film on a surface. Another possibility is to create the polymer film by a spraying, etc.

The biocompatible polymer composition according to the invention is ideally used for a medical device intended to contact blood, tissue, skin, epithelial layers, wounds, cells in culture fluids, body fluids, dialysis fluids and/or therapeutic fluids for removal or infusion in order to enhance biocompatibility and prevent bacterial growth, preferably a catheter to get transcutanous access to the body of a patient including peritoneal catheter including patient extension lines, trancutaneous tunnels, e g cuffs, but it could also be used in a dialysis monitor wherein the composition may be used to coat the lines wherein the dialysis fluid is generated. Other fields of application are infusions therapy, implantation technology, intravenous nutrition, urethral catheter, etc.

Further, it is also possible to advantageously use the disclosed invention in any technical system, e.g. water processing systems, water pipe systems, in air filters, in membrane based separation systems to prevent fouling process, in biosensors, wound dressing or wound coverage substrate media, bioreactors, in food processing systems where biofilm formation should be prevented and biocompatibility and non-toxicity is of critical importance. Other fields of application where the described properties have obvious advantageous are sanitary products, skin or food care products including wound dressing, surgical instruments, endoscopes, textiles, hygiene articles, such as toothbrushes, wound care products, plasters, tamponates, stoma bags, storage containers, refrigerators (e.g. for storage of drugs, medical products or food) etc.

The present invention will now be illustrated by way of non-limiting examples of preferred embodiments in order to further facilitate the understanding of the invention.

### Examples

### Example 1:

### Film preparation

Step 1:
   60 g Methylisobutylketone.
   5 g TEGOMER H-Si 6440 (Goldschmidt A. G.)
   Warm up to 50°C under light stirring for approximately 5 min.
   TEGOMER H-Si 6440 is a triblock copolymer of polycaprolactone-polydimethylsiloxane-polycaprolactone blocks having nominal molecular weights of 2000, 2000 and 2000, respectively.
Step 2:
   Add 0,01-0,32 g triphenylbismuth dichloride (511.21 g/mol) (Aldrich), corresponding to 0,004-0,13 g bismuth.
   Light stirring at room temperature for approximately 5 min.
Step 3:
   Add 35 g DESDOMUR E23 (Bayer Co.).
   Light stirring to avoid air bubble formation.
   Degassing is required to remove air bubbles.
   DESMODUR E23 is a polyisocyanate prepolymer based on diphenyl methane diisocyanate. The -NCO content is 15,4 weight%. Equivalent weight is 273.
Step 4:
   a) Casting a film in various thickness on glass plate with or without support foil, e. g. PE (polyethylene or injection moulded plates made from polyurethane); or
   b) Film forming by transporting solution outside and through catheter tubes (ID 1-3 mm or any other geometry).
      The polymerised film was then examined by scanning electron microscopy.

### Example 2

### Testing/assessing thrombogenicity

PUR plates and films with or without triphenylbismuth dichloride (the ones with triphenylbismuth dichloride is the same as is described in example 1) were tested for thrombogenicity assessment using freshly donated human blood. During contact of blood components with the material the kinetic generation of thrombin-anti-thrombin III complex (TAT) was analysed as an indicator of thrombin formation. Thrombin is the major component in the coagulation circuit, since thrombin is a potent activator for platelets and cleaves fibrinogen to fibrin which finally leads to a polymerised fibrin network, i. e. a clot. TAT was measured by a commercially available ELISA test according to the instruction of the manufacturer (Behring Co., Germany). The comparison of materials/surfaces is done in direct comparison of the modified versus the non-modified polymer system. Accelerated reaction kinetics for TAT indicates less biocompatible, more thrombogenic material.

For details on methodology for thromogenicity assessment: Deppisch R. et al (1993) Nephrol. Dial. Transplant Supp. 3 (1994) 17-23 and Tsai et al (1994) ASAIO J. 40:M619-M624.

In vitro analysis was performed with freshly donated human whole blood. TAT data after 40 min blood contact for TEGOMER-PUR-Bi films prepared according to Example 1 are shown in table 1.

**Table 1: TAT values after 40 min activation with human whole blood**

| Material | TAT[µg/l] |
|---|---|
| Uncoated plate PUR(Tecoflex) | 363 |
| Film PUR -TEGOMER | 210 |
| Film PUR -TEGOMER-0.03%Bi | 224 |
| Positive control | >2000 |

Data depicted in table 1 show that films on surfaces result in reduction of thrombin formation in whole blood compared to non-treated standard PUR surfaces (polyurethane formulation by Thermedics Co., Tecoflex^{®}, is standard polymer material in hemodialysis catheters). There is no negative influence by adding the bismuth complex triphenylbismuth dichloride compared to films without bismuth complex. These experiments were performed versus a positive control which is polystyrene (as used in Greiner tissue culture plates) resulting in a TAT formation of >2000 µg/l.

### Example 3

### Cell toxicity studies

The toxicity of various combinations of film coatings prepared according to example 1 was evaluated by measuring inhibition of cell growth (ICG). ICG was measured by making aqueous eluates of the various test materials, then incubating growing mammalian cells in culture medium containing the eluate, and then evaluating the cell viability by neutral red uptake.

The ICG test was begun by seeding a 96-well tissue culture plate with 1500-2000 mouse fibroblast cells (strain L-929) previously grown to subconfluence for 48-72 h in complete Eagles MEM (minimal essential media as described in text books for cell culture). The plates were incubated for 24 h at 37°C. The medium was then removed and test eluates were added and incubated. The test eluates were made by incubating test plates or films in distilled water (1 ml for each 10 cm² test material) at 70°C for 24 h.

For each plate, 250 µl 0.4% neutral red solution was mixed with 20 ml of complete Eagle's MEM. The eluate incubation medium was removed and 200 µl/well of neutral red containing medium was added. The plates were then incubated for 3 h at 37°C. The solution was then discarded, the plates rinsed with 200 µl PBS/well. After that, 200 µl/well of 50% (v/v) ethanol and 1% (v/v) acetic acid in distilled water was added. After a 10 min wait the absorbance at 540 nm of each well was measured. ICG% was calculated as (Aₖ-A_{T})/Aₖx100, where AT=mean absorbance in test solution minus mean absorbance in blank. The materials are deemed non-toxic if ICG is < 30%, as described by Wieslander et al (1991) Kidney International 49:77.79.

The following materials were employed:
Completed Eagles MEM:
500 ml Eagles MEM
50 ml Fetal calf serum

5 ml 200 m ML-Glutamine
5 ml Non-Essential Amino Acid solution
0.5 ml Gentamycin 50 mg/ml

| PBS (10xstock solution) | |
|---|---|
| NaCl | 80 g |
| KCL | 2 g |
| KH₂PO 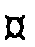 | 2 g |
| Na₂HPO₄·H₂O | 11 g |

Dissolve in H₂O to 1000 ml final volume.

The stock solution is diluted 10-fold and pH adjusted to 7.2

50% ethanol, 1% acetic acid solution:
500 ml ethanol (96%)
490 ml water
   10 ml Glacial acetic acid
   4% Neutral red stock solution:
4 g Neutral red (Merck No. 1376)
100 ml distilled water
   Diluted 10-fold with water prior to use.

The results of the investigated films (prepared according to Example 1) are shown in Table 2.

**Table 2: ICG levels of PUR films (example 1)**

| Film | ICG (%) |
|---|---|
| PUR | 5 |
| PUR-TEGOMER | 3 |
| PUR-TEGOMER-0.03%Bi | 3 |
| PUR-TEGOMER-0.08%Bi | 2 |
| PUR-TEGOMER-0.24%Bi | 97 |
| PUR-TEGOMER-0.32%Bi | 92 |

Films (5-20 µm thick) with a triphenylbismuth dichloride concentration of up to 0.2 weight%, corresponding to 0.08 weight% bismuth were non-toxic in ICG assay. Films with 0.6 weight% triphenylbismuth dichloride, corresponding to 0.24 weight% bismuth and more were toxic.

As depicted in the table, inhibition of cell growth can only be seen in concentrations of bismuth > 0.2%. These results together with the thrombogenicity show that bismuth as an additive component for the polyurethane PL-PDMS-PL formulation has an effect on reduced formation of thrombin and no toxicity in low concentrations (< 0.08% Bi). This could lead to a reduced risk for thrombus formation and clot deposits in clinical circumstances and by this advantageously address or limit the related events of clotting followed by bacterial growth or vice versa, as it is known that clot layers, i.e.- fibrin net work with entrapped platelets or other blood cells, provide a good substrate for bacteria adhesion and biofilm development.

### Example 4

### Bacterial adhesion

Bacterial adhesion was tested by two different methodologies, with the MTT assay and by scanning electron microscopy of bacterial growth. The MTT test is a rapid and sensitive colorimetric assay based on the formation of a coloured insoluble formazan salt. The amount of formazan produced is directly proportional to the cell number and therefore can be used to measure cell viability and proliferation. The assay is based on the capacity of the mithocondrial dehydrogenase enzymes to convert yellow water-soluble tetrazolium salt (=MTT) into a purple insoluble formazan product by a reduction reaction. These insoluble crystals are dissolved in DMSO and the absorbance is read with a spectrophotometer at 550-570 nm.

The MTT test was begun by seeding a concentration of 10⁵/ml of Staphylococcus epidermidis (ATCC 12228) in a trypcase-soja bouillon into a 24-well plates with different films and were incubated in 4 h, 8 h, 24 h, 48 h, at 37°C. After incubation the bouillon was removed and the plates were washed with PBS-buffer. Then 500 µl/well MTT solution (0.5 mg/ml in PBS) was added and incubated for another 30 minutes at 37°C. The solution was removed and 500 µl/well lysis solution (99.4 ml DMSO; 0.6 ml 100% glacial acetic acid; 10 g SDS) was added. After 10 min incubation on microtiter shaker the solution was pipetted into a 96-well plate and the absorbance was measured at 55 nm (against reference of 620 nm)

The following materials were employed:
Staphyloccus epidermis ATCC 12228
Plate-count-agar
Trypcase-soja bouillon
PBS buffer: 8.0 g NaCl
   0.2 g KCl
   1.44 g Na2HPO4 x 2 H2O
   0.2 g KH2PO4
   dissolve in 1000 ml distilled water; pH 7.2
   MTT solution (0.5 mg/ml in PBS)
   Lysis solution (99.4 ml DMSO; 0.6 ml glacial acetic acid; 10 g SDS)

It could be clearly shown that the addition of a bismuth complex in the polymer formulation leads to complete inhibition of bacteria proliferation as measured by MTT (Table 3). It is most important that addition of bismuth in low concentration, e g non-toxic, for example 0.03 %, the inhibition of bacteria proliferation cannot be correlated with the cytotoxicity of extracts.

**Table 3: Results of the MTT test (mean value of two experiments)**

| Extinction (nm) | | | |
|---|---|---|---|
| | 24 h | 48 h | 72 h |
| Catheter material (Tecoflex) | 0.39 | 0.51 | 0.36 |
| Film PUR | 0.47 | 0.31 | 0.34 |
| Film PUR-TEGOMER | 0.40 | 0.38 | 0.29 |
| Film PUR-0.32%Bi | 0 | 0 | |
| Film PUR-TEGOMER-0.32%Bi | 0 | 0 | |
| Film PUR-TEGOMER-0.08%Bi | 0 | 0 | |
| Film PUR-TEGOMER-0.03%Bi | 0 | 0 | |

By another method electron microscopy of bacterial growth on bismuth containing PL-PDMS-PL PUR-polymeric films was performed. By these experiments it can be clearly confirmed that no bacterial growth could be detected on the Bi modified surfaces over a period of time of 32 h. No bacteria colonization or biofilm formation occurred.

### Example 5

### Bismuth surface concentration (XPS)

In order to assess the presence of bismuth on the surface x-ray fluorescence spectroscopy (XPS) was applied. Results for films prepared as in example 1 were received for different take-off angles (TOA), 10° and 90°. The greater the take off angle the higher the penetration depth for this analysis. The data show that bismuth can be detected on the surface of the films containing triphenylbismuth dichloride. The concentration is close to the detection limit of bismuth with XPS.

**Table 4: Bismuth concentration /atom (%) on the surface measured by XPS.**

| Film | Bismuth atom (%) | Bismuth atom(%) |
|---|---|---|
| | TOA 90° | TOA 10° |
| PUR-TEGOMER | <0.001 | <0.001 |
| PUR-TEGOMER-0.08%Bi | 0.006 | 0.006 |
| PUR-TEGOMER- 0.32%Bi | 0.02 | 0.02 |
| PUR-0.32%Bi | 0.01 | 0.02 |

To further characterize the materials of the invention analyses of bismuth surface concentrations were performed on the polymeric films. In films containing 0.08% bismuth 0.006 atom% was discovered on the surface. This should be the maximum concentration active in preventing bacteria growth and biofilm formation. This bismuth concentration is extremely low and even so surprisingly effective.

### Example 7

### Bismuth in aqueous eluate

The films were further characterized by their ability to release bismuth, see table. It could be shown that these films release in aqueous environment 0.02 mg/l extraction fluid which is below 0.05% of the total amount of bismuth given to the polymer formulation used for 600 cm² bismuth containing polymer film.

**Table 5: Bismuth concentration in aqueous eluates**

| Eluates of Films (5-20µm) | Bi [mg/l] |
|---|---|
| PUR-TEGOMER | 0 |
| PUR-TEGOMER-0.03%Bi | 0.02 |
| PUR-TEGOMER-0.05%Bi | 0.03 |
| PUR-TEGOMER-0.08%Bi | 0.03 |
| PUR-TEGOMER-0.24%Bi | 0.19 |

### Example 8

### Bismuth after extraction with human whole blood

Coated catheters were tested by their ability to release bismuth in human blood. Therefore 50 ml citrated/heparinised whole blood were used for the extraction of each catheter. The extraction was made by 6 h recirculation and 24 h in the incubator to simulate a cycle of treatment period and inter-treatment intravascular position. For measurement the samples were treated with nitric acid to release Bi from the blood component matrix and measured with AAS within the precision of the analytic tools. The measurable amount of bismuth in whole blood was almost the same for bismuth-SMA coated catheters (mean valus of 3 catheters) as for standard catheter (without coating). There is no enrichment of bismuth measurable in blood.

**Table 6: Whole blood bismuth concentration after extraction**

| Catheter | After 0h Bi (mg/l) | After 6 h recirculation, 37°C Bismuth mg/ml |
|---|---|---|
| Control (whole blood after blood donation) | 0.005 | |
| PUR (Tecoflex) non-coated catheter | | 0.015 |
| PUR-Tegomer-0,03 % Bi coating (mean of 3 catheters) | | 0.014 |

### Example 9

### Film preparation

Step 1:
   80g Methylisobutylketone
   20g Silicon MED 1011 (Nusil, Polytec GmbH)
   Mixing under light stirring at room temperature for app. 20 min. MED-1011 is a one component, self leveling silicone.
Step2:
   Add 0,03g triphenylbismuth dichloride (511.21g/mol) (Aldrich) corresponding to 0,01g bismuth. Light stirring at room temperature for app. 10 min.
      a. Casting a film on a glass plate with support foil e.g. polyethylene
      b. Film forming by transporting the solution outside and through a silicon catheter tube

The film was examined by scanning electron microscopy.

### Example 10:

### Bacterial adhesion

Bacterial adhesion and proliferation was measured with the BacTrac System (Sy-Lab GmbH, Austria). With this impedance method the change of ionic composition of the nutrient media caused by the microbial metabolism is used as parameter wherein the sum of all metabolism outputs is continuously detected. The change in impedance correlates with amount of proliferating bacteria on the sample.

This method is described by Futschik et al (1995): Electrode and Media Impedance for the Detection and Characterisation of Microorganisms. Proceedings RC IEEE-EMBS & 14th BMESI, 1.75-1.76.

For measurement of bacterial adhesion and proliferation films prepared as in Example 9 were incubated with a concentration of 30*10⁶ Staphylococcus epidermidis in a trypcase-soja boullion for 24h hours at 37°C.

Subsequently, the pre-incubated films were transferred into the measuring cell of the BacTrac system filled with fresh boullion. Impedance measurement with BacTrac was done over 20h.

It could be shown that the addition of bismuth in the silicon polymer formulation leads to an inhibition/delay of proliferation as already shown for the PUR/SMA-bismuth formulations.

### Example 11:

### ICG levels of the silicon- bismuth films:

Example 11 was performed on the silicon-bismuth films prepared in Example 9 in accordance with Example 3.

The results of the investigated films (prepared according to Example 9) are shown in Table 7.

**Table 7: ICG levels of silicon-bismuth films (Example 9)**

| Film | ICG (%) |
|---|---|
| Silicon (without bismuth) | 0,3 |
| Silicon 0,06% bismuth | 0 |
| Silicon 0,2% bismuth | 99,6 |

Films (5-20 µm thick) with a triphenylbismuth dichloride concentration of 0,5weight % , corresponding to 0,2 weight % bismuth are toxic in the ICG assay. Silicon films without bismuth and films with 0,15 weight % triphenylbismuth dichloride, corresponding to 0,06% bismuth show no inhibition of cell growth.

## Claims

1. A biocompatible polymer composition for an article having a surface intended to contact blood, tissue, skin, epithelial layers, wounds, cells in culture fluids, body fluids, dialysis fluids and/or therapeutic fluids for removal or infusion, **characterized in that** the polymer composition comprises a bismuth complex incorporated into the polymer composition in an amount corresponding to 0.002 to 0.08 weight% bismuth of the polymer composition.

2. A biocompatible polymer composition according to claim 1, **characterized in that** said polymer composition further comprises nano-particles, which include a bismuth complex.

3. A biocompatible polymer composition according to claim 1 or 2, **characterized in that** the bismuth complex is chosen from the group comprising ammonium bismuth citrate, bismuth (III) oxide, bismuth (III) gallate hydrate, bismuth citrate, bismuth (III) oxychloride, bis- muth (III) tetramethylheptanedionate, bismuth (III) hexafluoracetonate, bismuth (III) subsalicylate, triphenylbismuth, bismuth (III) ciprofloxacin, bismuth (III)-chloride, triphenylbismuth dichloride, triphenylbismuth carbonate, triphenylbismuth dihydroxide, triphenylbismuth dinitrate, triphenylbismuth disalicylate, triphenylbismuthine, triphenylbismuth bis (2- chloroacetate), triphenylbismuth bis (4-aminobenzoate), bis(acetato-O) triphenylbismuth, dibromotriphenylbismuth, and difluorotriphenylbismuth.

4. A biocompatible polymer composition according to claim 1 or 2, **characterized in that** the bismuth complex is triphenylbismuth or triphenylbismuth dichloride, preferably triphenylbismuth dichloride.

5. A biocompatible polymer composition according to any of claims 1-3, **characterized in that** the polymer composition comprises a film-forming polymer component.

6. A biocompatible polymer composition according to claim 5, **characterized in that** the film-forming polymer is a siloxane polymer and/or a siloxane oligomer.

7. A biocompatible polymer composition according to claim 5, **characterized in that** the polymer composition further comprises a hydrophobic polymer block having reactive end groups.

8. A biocompatible polymer composition according to claim 7, **characterized in that** the film-forming polymer is selected from the group of polyurethanes, polyolefins, elastomers, polyethylene- glycols, polycarbonates, polyethersulphones, polysulfones, polyvinyl pyrrolidones, and polyvinylchlorides, and the hydrophobic block is selected from the group of siloxane polymers, siloxane oligomers, fluoropolymers, polyethyleneglycols, polyethyleneglycol-polydimethyl siloxane copolymers, silicone polyesters, and polylactone-polysiloxane-polylactone triblock copolymers.

9. A biocompatible polymer composition according to claim 8, **characterized in that** the film-forming polymer is a polyurethane and the hydrophobic polymer block is a polylactone-polysiloxane-polylactone triblock copolymer containing mono, bi or multiple reactive end groups.

10. A method for the preparation of a biocompatible copolymers composition, **characterri-zed** in that a bismuth complex is incorporated into the polymer composition in an amount corresponding to 0.002 to 0.08 weight% bismuth of the polymer composition.

11. A method for preparation of a biocompatible copolymer composition according to claim 10, **characterized in that** nano-particles containing a bismuth complex are incorporated into the polymer composition.

12. A method for the preparation of a biocompatible copolymer composition according to claim 10 or 11, **characterized in that** the bismuth complex is chosen from the group comprising ammonium bismuth citrate, bismuth (III) oxide, bismuth (III)-gallate hydrate, bismuth citrate, bismuth (III) oxychloride, bismuth (III) tetramethylheptanedionate, bismuth (III) hexafluoracetonate, bismuth (III) subsalicylate, triphenylbismuth, bismuth (III) ciprofloxacin, bismuth (III) chloride, triphenylbismuth dichloride, triphenylbismuth carbonate, triphenylbismuth dihydroxide, triphenylbismuth dinitrate, triphenylbismuth disalicylate, triphenylbismuthine, triphenylbismuth bis (2-chloroacetate), triphenylbismuth bis (4-aminobenzoate), bis(acetato-O) triphenylbismuth, dibromotriphenylbismuth, and difluorotriphenylbismuth.

13. A method for the preparation of a biocompatible copolymer composition according to claim 10 or 11, **characterized in that** the bismuth complex is triphenylbismuth or triphenylbismuth dichloride, preferably triphenylbismuth dichloride.

14. A method for the preparation of a biocompatible copolymer composition according to any one of claims 10-13, **characterized in** the steps of combining a film-forming polymer composition and a bismuth complex in the presence of a solvent such that all components are dissolved in the solvent, incubating the components dissolved in the solvent under conditions to allow the components to react with one another in solution, and forming a film by spreading the solution over a surface to be coated under conditions that allow the solvent to evaporate.

15. A method for the preparation of a biocompatible copolymer composition according to any of claims 10-14, **characterized in that** the film-forming polymer is a siloxane polymer and/or a siloxane oligomer and the bismuth complex a triphenylbismuth dichloride.

16. A method for the preparation of a biocompatible copolymer composition according to any one of claims 10-13, **characterized in** the steps of combining a film-forming polymer composition and a bismuth complex with a hydrophobic polymer block having end groups reactive with the film-forming polymer component in the presence of a solvent such that all components are dissolved in the solvent, incubating the components dissolved in the solvent under conditions to allow the components to react with one another in solution, and forming a film by spreading the solution over a surface to be coated under conditions that allow the solvent to evaporate.

17. A method for the preparation of a biocompatible copolymer composition according to any of claims 10-13 and 16, **characterized in that** the film-forming polymer is a polyurethane, the bismuth complex a triphenylbismuth dichloride and the hydrophobic polymer block is a polylactone-polysiloxane-polylactone triblock copolymer containing mono, bi or multiple reactive end-groups.

18. An article having a surface intended to contact blood, tissue, skin, epithelial layers, wounds, cells in culture fluids, body fluids, dialysis fluids
and/or therapeutic fluids for removal or infusion, **characterized in that** said article has at least one film of a polymer composition comprising a bismuth complex in an amount corresponding to 0.002 to 0.08 weight% bismuth of the polymer composition, covering said surface.

19. An article according to claim 18, **characterized in that** said article has a multilayer coating of said polymer composition.

20. An article according to claim 18, **characterized in that** said article has a coating of said polymer composition and that said polymer composition further include nano-particles, which include a bismuth complex.

21. An article according to claim 20, **characterized in that** said polymer composition comprises a film-forming polymer component.

22. An article according to claim 21, **characterized in that** the film-forming polymer is a siloxane polymer and/or a siloxane oligomer.

23. An article according to claim 21, **characterized in that** said polymer composition further comprises a hydrophobic polymer block having one or more reactive end groups.

24. An article according to claim 23, **characterized in that** the film-forming polymer is selected from the group of polyurethanes, polyolefins, elastomers, polyethyleneglycols, polycarbonates, polyethersulphones, polysulfones, polyvinyl pyrrolidones, and polyvinylchlorides, and the hydrophobic block is selected form the group of siloxane polymers, siloxane oligomers, fluoropolymers, polyethyleneglycols, polyethyleneglycol-polydimethyl siloxane copolymers, silicone polyesters, and polylactone-polysiloxane- polylactone triblock copolymers.

25. An article according to claim 24, **characterized in that** the film-forming polymer is a polyurethane and the hydrophobic polymer block is a polylactone-polysiloxane-polylactone triblock copolymer containing mono, bi or multiple reactive endgroups.

26. Use of a biocompatible polymer composition according to claim 1 for a medical device intended to contact blood, tissue, tissue with wounds, cells in culture fluids, body fluids, dialysis fluid or therapeutic fluids for removal or infusion in order to enhance biocompatibility and prevent bacterial growth.

27. Use of a biocompatible polymer composition according to claim 1 on surfaces in contact with blood, tissue, skin, epithelial layers, wounds, cells in culture fluids, body fluids, dialysis fluids and/or therapeutic fluids for removal or infusion.

28. A biocompatible polymer composition according to claim 9, **characterized in that** the release of bismuth from said polymer composition into an aqueous solution is below 0.05% of the total amount of bismuth contained in the polymer composition.

29. An article according to claim 18, wherein the at least one film consists of a biocompatible polymer composition according to claim 9, **characterized in that** the release of bismuth from the at least one film into an aqueous solution is below 0.05% of the total amount of bismuth contained in the polymer composition.

## Patentansprüche

1. Biokompatible Polymerzusammensetzung für einen Gegenstand mit einer Oberfläche, die dazu vorgesehen ist, mit Blut, Gewebe, Haut, Epithelschichten, Wunden, Zellen in Kulturfluiden, Körperfluiden, Dialysefluiden und/oder therapeutischen Fluiden zur Beseitigung oder Infusion in Kontakt zu geraten, **dadurch gekennzeichnet, dass** die Polymerzusammensetzung einen Bismutkomplex umfasst, der in der Polymerzusammensetzung in einer Menge eingebaut ist, die 0,002 bis 0,08 Gew.% Bismut der Polymerzusammensetzung entspricht.

2. Biokompatible Polymerzusammensetzung nach Anspruch 1, **dadurch gekennzeichnet, dass** die Polymerzusammensetzung ferner Nanoteilchen umfasst, die einen Bismutkomplex umfassen.

3. Biokompatible Polymerzusammensetzung nach Anspruch 1 oder 2, **dadurch gekennzeichnet, dass** der Bismutkomplex aus der Ammoniumbismutcitrat, Bismut(III)-oxid, Bismut(III)-gallathydrat, Bismutcitrat, Bismut(III)-oxichlorid, Bismut(III)-tetramethylheptandionat, Bismut(III)-hexafluoracetonat, Bismut(III)-subsalicylat, Triphenylbismut, Bismut(III)-ciprofloxacin, Bismut(III)-chlorid, Triphenylbismutdichlorid, Triphenylbismutcarbonat, Triphenylbismutdihydroxid, Triphenylbismutdinitrat, Triphenylbismutdisalicylat, Triphenylbismutin, Triphenylbismut-bis(2-chloracetat), Triphenylbismut-bis(4-aminobenzoat), Bis(acetato-O)triphenylbismut, Dibromtriphenylbismut und Difluortriphenylbismut umfassenden Gruppe ausgewählt ist.

4. Biokompatible Polymerzusammensetzung nach Anspruch 1 oder 2, **dadurch gekennzeichnet, dass** der Bismutkomplex Triphenylbismut oder Triphenylbismutdichlorid, vorzugsweise Triphenylbismutdichlorid, ist.

5. Biokompatible Polymerzusammensetzung nach einem der Ansprüche 1 bis 3, **dadurch gekennzeichnet, dass** die Polymerzusammensetzung eine filmbildende Polymerkomponente umfasst.

6. Biokompatible Polymerzusammensetzung nach Anspruch 5, **dadurch gekennzeichnet, dass** das filmbildende Polymer ein Siloxanpolymer und/oder ein Siloxanoligomer ist.

7. Biokompatible Polymerzusammensetzung nach Anspruch 5, **dadurch gekennzeichnet, dass** die Polymerzusammensetzung ferner einen hydrophoben Polymerblock mit reaktiven Endgruppen umfasst.

8. Biokompatible Polymerzusammensetzung nach Anspruch 7, **dadurch gekennzeichnet, dass** das filmbildende Polymer aus der Gruppe der Polyurethane, Polyolefine, Elastomere, Polyethylenglykole, Polycarbonate, Polyethersulfone, Polysulfone, Polyvinylpyrrolidone und Polyvinylchloride ausgewählt ist und der hydrophobe Block aus der Gruppe der Siloxanpolymere, Siloxanoligomere, Fluorpolymere, Polyethylenglykole, Polyethylenglykol-Polydimethylsiloxan-Copolymere, Siliconpolyester und Polylacton-Polysiloxan-Polylacton-Triblockcopolymere ausgewählt ist.

9. Biokompatible Polymerzusammensetzung nach Anspruch 8, **dadurch gekennzeichnet, dass** das filmbildende Polymer ein Polyurethan ist und der hydrophobe Polymerblock ein Polylacton-Polysiloxan-Polylacton-Triblockcopolymer ist, das mono-, bi- oder multiple reaktive Endgruppen enthält.

10. Verfahren zur Herstellung einer biokompatiblen
Copolymerzusammensetzung, **dadurch gekennzeichnet, dass** ein Bismutkomplex in der Polymerzusammensetzung in einer Menge, die 0,002 bis 0,08 Gew.% Bismut der Polymerzusammensetzung entspricht, eingebaut ist.

11. Verfahren zur Herstellung einer biokompatiblen Copolymerzusammensetzung nach Anspruch 10, **dadurch gekennzeichnet, dass** Nanoteilchen, die einen Bismutkomplex enthalten, in der Polymerzusammensetzung eingebaut sind.

12. Verfahren zur Herstellung einer biokompatiblen Copolymerzusammensetzung nach Anspruch 10 oder 11, **dadurch gekennzeichnet, dass** der Bismutkomplex aus der Ammoniumbismutcitrat, Bismut(III)-oxid, Bismut(III)-gallathydrat, Bismutcitrat, Bismut(III)-oxichlorid, Bismut(III)-tetramethylheptandionat, Bismut(III)-hexafluoracetonat, Bismut(III)-subsalicylat, Triphenylbismut, Bismut(III)-ciprofloxacin, Bismut(III)-chlorid, Triphenylbismutdichlorid, Triphenylbismutcarbonat, Triphenylbismutdihydroxid, Triphenylbismutdinitrat, Triphenylbismutdisalicylat, Triphenylbismutin, Triphenylbismut-bis(2-chloracetat), Triphenylbismut-bis(4-aminobenzoat), Bis(acetato-O)triphenylbismut, Dibromtriphenylbismut und Difluortriphenylbismut umfassenden Gruppe ausgewählt ist.

13. Verfahren zur Herstellung einer biokompatiblen Copolymerzusammensetzung nach Anspruch 10 oder 11, **dadurch gekennzeichnet, dass** der Bismutkomplex Triphenylbismut oder Triphenylbismutdichlorid, vorzugsweise Triphenylbismutdichlorid, ist.

14. Verfahren zur Herstellung einer biokompatiblen Copolymerzusammensetzung nach einem der Ansprüche 10 bis 13, **gekennzeichnet durch** die Schritte des Kombinierens einer filmbildenden Polymerzusammensetzung und eines Bismutkomplexes in der Gegenwart eines Lösungsmittels, sodass alle Komponenten im Lösungsmittel gelöst werden, des Inkubierens der im Lösungsmittel gelösten Komponenten unter Bedingungen, die es zulassen, dass die Komponenten in Lösung miteinander reagieren, und des Bildens eines Films **durch** Verteilen der Lösung auf einer zu beschichtenden Oberfläche unter Bedingungen, die es zulassen, dass das Lösungsmittel verdampft.

15. Verfahren zur Herstellung einer biokompatiblen Copolymerzusammensetzung nach einem der Ansprüche 10 bis 14, **dadurch gekennzeichnet, dass** das filmbildende Polymer ein Siloxanpolymer und/oder ein Siloxanoligomer und der Bismutkomplex ein Triphenylbismutdichlorid ist.

16. Verfahren zur Herstellung einer biokompatiblen Copolymerzusammensetzung nach einem der Ansprüche 10 bis 13, **gekennzeichnet durch** die Schritte des Kombinierens einer filmbildenden Polymerzusammensetzung und eines Bismutkomplexes mit einem hydrophoben Polymerblock mit Endgruppen, die mit der filmbildenden Polymerkomponente reaktiv sind, in der Gegenwart eines Lösungsmittels, sodass alle Komponenten im Lösungsmittel gelöst werden, des Inkubierens der im Lösungsmittel gelösten Komponenten unter Bedingungen, die es zulassen, dass die Komponenten in Lösung miteinander reagieren, und des Bildens eines Films **durch** Verteilen der Lösung auf einer zu beschichtenden Oberfläche unter Bedingungen, die es zulassen, dass das Lösungsmittel verdampft.

17. Verfahren zur Herstellung einer biokompatiblen Copolymerzusammensetzung nach einem der Ansprüche 10 bis 13 und 16, **dadurch gekennzeichnet, dass** das filmbildende Polymer ein Polyurethan ist, der Bismutkomplex ein Triphenylbismutdichlorid ist und der hydrophobe Polymerblock ein Polylacton-Polysiloxan-Polylacton-Triblockcopolymer ist, das mono-, bi- oder multiple reaktive Endgruppen enthält.

18. Gegenstand mit einer Oberfläche, die dazu vorgesehen ist, mit Blut, Gewebe, Haut, Epithelschichten, Wunden, Zellen in Kulturfluiden, Körperfluiden, Dialysefluiden und/oder therapeutischen Fluiden zur Beseitigung oder Infusion in Kontakt zu geraten, **dadurch gekennzeichnet, dass** der Gegenstand zumindest einen Film, der die die Oberfläche bedeckt, aus einer Polymerzusammensetzung aufweist, die einen Bismutkomplex in einer Menge umfasst, die 0,002 bis 0,08 Gew.% Bismut der Polymerzusammensetzung entspricht.

19. Gegenstand nach Anspruch 18, **dadurch gekennzeichnet, dass** der Gegenstand eine Mehrschichtbeschichtung aus der Polymerzusammensetzung aufweist.

20. Gegenstand nach Anspruch 18, **dadurch gekennzeichnet, dass** der Gegenstand eine Beschichtung aus der Polymerzusammensetzung aufweist und dass die Polymerzusammensetzung ferner Nanoteilchen umfasst, die einen Bismutkomplex umfassen.

21. Gegenstand nach Anspruch 20, **dadurch gekennzeichnet, dass** die Polymerzusammensetzung eine filmbildende Polymerkomponente umfasst.

22. Gegenstand nach Anspruch 21, **dadurch gekennzeichnet, dass** das filmbildende Polymer ein Siloxanpolymer und/oder ein Siloxanoligomer ist.

23. Gegenstand nach Anspruch 21, **dadurch gekennzeichnet, dass** die Polymerzusammensetzung ferner einen hydrophoben Polymerblock mit einer oder mehreren reaktiven Endgruppen umfasst.

24. Gegenstand nach Anspruch 23, **dadurch gekennzeichnet, dass** das filmbildende Polymer aus der Gruppe der Polyurethane, Polyolefine, Elastomere, Polyethylenglykole, Polycarbonate, Polyethersulfone, Polysulfone, Polyvinylpyrrolidone und Polyvinylchloride ausgewählt ist und der hydrophobe Block aus der Gruppe der Siloxanpolymere, Siloxanoligomere, Fluorpolymere, Polyethylenglykole, Polyethylenglykol-Polydimethylsiloxan-Copolymere, Siliconpolyester und Polylacton-Polysiloxan-Polylacton-Triblockcopolymere ausgewählt ist.

25. Gegenstand nach Anspruch 24, **dadurch gekennzeichnet, dass** das filmbildende Polymer ein Polyurethan ist und der hydrophobe Polymerblock ein Polylacton-Polysiloxan-Polylacton-Triblockcopolymer ist, das mono-, bi- oder multiple reaktive Endgruppen enthält.

26. Verwendung einer biokompatiblen Polymerzusammensetzung nach Anspruch 1 für eine medizinische Vorrichtung, die dazu vorgesehen ist, mit Blut, Gewebe, Gewebe mit Wunden, Zellen in Kulturfluiden, Körperfluiden, Dialysefluiden oder therapeutischen Fluiden zur Beseitigung oder Infusion in Kontakt zu geraten, zur Verbesserung der Biokompatibilität und Verhinderung von Bakterienwachstum.

27. Verwendung einer biokompatiblen Polymerzusammensetzung nach Anspruch 1 auf Oberflächen, die in Kontakt mit Blut, Gewebe, Haut, Epithelschichten, Wunden, Zellen in Kulturfluiden, Körperfluiden, Dialysefluiden und/oder therapeutischen Fluiden zur Beseitigung oder Infusion sind.

28. Biokompatible Polymerzusammensetzung nach Anspruch 9, **dadurch gekennzeichnet, dass** die Freigabe von Bismut aus der Polymerzusammensetzung in eine wässrige Lösung weniger als 0,05 % der Gesamtmenge an Bismut, die in der Polymerzusammensetzung enthalten ist, beträgt.

29. Gegenstand nach Anspruch 18, wobei der zumindest eine Film aus einer biokompatiblen Polymerzusammensetzung nach Anspruch 9 besteht, **dadurch gekennzeichnet, dass** die Freigabe von Bismut aus dem zumindest einen Film in eine wässrige Lösung weniger als 0,05 % der Gesamtmenge an Bismut, die in der Polymerzusammensetzung enthalten ist, beträgt.

## Revendications

1. Composition polymère biocompatible pour un article ayant une surface destinée à être en contact avec le sang, le tissu, la peau, les couches épithéliales, les plaies, les cellules dans des liquides de culture, les fluides biologiques, les fluides de dialyse et/ou fluides thérapeutiques destinés à être éliminés ou à être perfusés, **caractérisée en ce que** la composition polymère comprend un complexe de bismuth incorporé dans la composition polymère en une quantité correspondant à 0,002 à 0,08 % en poids de bismuth de la composition polymère.

2. Composition polymère biocompatible selon la revendication 1, **caractérisée en ce que** ladite composition polymère comprend en outre des nanoparticules, qui comprennent un complexe de bismuth.

3. Composition polymère biocompatible selon la revendication 1 ou 2, **caractérisée en ce que** le complexe de bismuth est choisi dans le groupe comprenant le citrate d'ammonium et de bismuth, l'oxyde de bismuth (III), le gallate de bismuth (III) hydraté, le citrate de bismuth, l'oxychlorure de bismuth (III), le tétraméthylheptanedionate de bismuth (III), l'hexafluoracétonate de bismuth (III), le sous-salicylate de bismuth (III), le triphénylbismuth, la ciprofloxacine de bismuth (III), le chlorure de bismuth (III), le dichlorure de triphénylbismuth, le carbonate de triphénylbismuth, le dihydroxyde de triphénylbismuth, le dinitrate de triphénylbismuth, le disalicylate de triphénylbismuth, la triphénylbismuthine, le bis (2-chloroacétate) de triphénylbismuth, le bis (4-aminobenzoate) de triphénylbismuth, le bis (acétato-O)triphénylbismuth, le dibromotriphénylbismuth, et le difluorotriphénylbismuth.

4. Composition polymère biocompatible selon la revendication 1 ou 2, **caractérisée en ce que** le complexe de bismuth est du triphénylbismuth ou du dichlorure de triphénylbismuth, de préférence du dichlorure de triphénylbismuth.

5. Composition polymère biocompatible selon l'une quelconque des revendications 1 à 3, **caractérisée en ce que** la composition polymère comprend un composant polymère filmogène.

6. Composition polymère biocompatible selon la revendication 5, **caractérisée en ce que** le polymère filmogène est un polymère de siloxane et/ou un oligomère de siloxane.

7. Composition polymère biocompatible selon la revendication 5, **caractérisée en ce que** la composition polymère comprend en outre une séquence polymère hydrophobe ayant des groupes terminaux réactifs.

8. Composition polymère biocompatible selon la revendication 7, **caractérisée en ce que** le polymère filmogène est choisi dans le groupe constitué par les polyuréthanes, les polyoléfines, les élastomères, les polyéthylèneglycols, les polycarbonates, les polyéthersulphones, les polysulfones, les polyvinylpyrrolidones, et les chlorures de polyvinyle, et la séquence hydrophobe est choisie dans le groupe constitué par les polymères de siloxane, les oligomères de siloxane, les fluoropolymères, les polyéthylèneglycols, les copolymères de polyéthylèneglycol-polydiméthyl siloxane, les polyesters de silicone, et les copolymères triséquencés de polylactone-polysiloxane-polylactone.

9. Composition polymère biocompatible selon la revendication 8, **caractérisée en ce que** le polymère filmogène est un polyuréthane et la séquence polymère hydrophobe est un copolymère triséquencé de polylactone-polysiloxane-polylactone contenant des groupes terminaux mono-, bi- ou multiréactifs.

10. Procédé de préparation d'une composition de copolymère biocompatible, **caractérisé en ce qu'**un complexe de bismuth est incorporé dans la composition polymère en une quantité correspondant à 0,002 à 0,08 % en poids de bismuth de la composition polymère.

11. Procédé de préparation d'une composition de copolymère biocompatible selon la revendication 10, **caractérisé en ce que** des nanoparticules contenant un complexe de bismuth sont incorporées dans la composition polymère.

12. Procédé de préparation d'une composition de copolymère biocompatible selon la revendication 10 ou 11, **caractérisé en ce que** le complexe de bismuth est choisi dans le groupe comprenant le citrate d'ammonium et de bismuth, l'oxyde de bismuth (III), le gallate de bismuth (III) hydraté, le citrate de bismuth, l'oxychlorure de bismuth (III), le tétraméthylheptanedionate de bismuth (III), l'hexafluoracétonate de bismuth (III), le sous-salicylate de bismuth (III), le triphénylbismuth, la ciprofloxacine de bismuth (III), le chlorure de bismuth (III), le dichlorure de triphénylbismuth, le carbonate de triphénylbismuth, le dihydroxyde de triphénylbismuth, le dinitrate de triphénylbismuth, le disalicylate de triphénylbismuth, la triphénylbismuthine, le bis (2-chloroacétate) de triphénylbismuth, le bis (4-aminobenzoate) de triphénylbismuth, le bis (acétato-O)triphénylbismuth, le dibromotriphénylbismuth, et le difluorotriphénylbismuth.

13. Procédé de préparation d'une composition de copolymère biocompatible selon la revendication 10 ou 11, **caractérisé en ce que** le complexe de bismuth est du triphénylbismuth ou du dichlorure de triphénylbismuth, de préférence du dichlorure de triphénylbismuth.

14. Procédé de préparation d'une composition de copolymère biocompatible selon l'une quelconque des revendications 10 à 13, **caractérisé par** les étapes consistant à combiner une composition polymère filmogène et un complexe de bismuth en présence d'un solvant de manière à ce que tous les composants soient dissous dans le solvant, à incuber les composants dissous dans le solvant dans des conditions qui permettent la réaction des composants les un avec les autres dans la solution, et à former un film par étalage de la solution sur une surface à revêtir dans des conditions qui permettent l'évaporation du solvant.

15. Procédé de préparation d'une composition de copolymère biocompatible selon l'une quelconque des revendications 10 à 14, **caractérisé en ce que** le polymère filmogène est un polymère de siloxane et/ou un oligomère de siloxane et le complexe de bismuth est un dichlorure de triphénylbismuth.

16. Procédé de préparation d'une composition de copolymère biocompatible selon l'une quelconque des revendications 10 à 13, **caractérisé par** les étapes consistant à combiner une composition polymère filmogène et un complexe de bismuth avec une séquence polymère hydrophobe ayant des groupes terminaux réactifs avec le composant polymère filmogène en présence d'un solvant de manière à ce que tous les composants soient dissous dans le solvant, à incuber les composants dissous dans le solvant dans des conditions qui permettent la réaction des composants les un avec les autres dans la solution, et à former un film par étalage de la solution sur une surface à revêtir dans des conditions qui permettent l'évaporation du solvant.

17. Procédé de préparation d'une composition de copolymère biocompatible selon l'une quelconque des revendications 10 à 13 et 16, **caractérisé en ce que** le polymère filmogène est un polyuréthane, le complexe de bismuth est un dichlorure de triphénylbismuth et la séquence de polymère hydrophobe est un copolymère triséquencé de polylactone-polysiloxane-polylactone contenant des groupes terminaux mono-, bi- ou multiréactifs.

18. Article ayant une surface destinée à être en contact avec le sang, le tissu, la peau, les couches épithéliales, les plaies, les cellules dans des liquides de culture, les fluides biologiques, les fluides de dialyse et/ou fluides thérapeutiques destinés à être éliminés ou à être perfusés, **caractérisé en ce que** ledit article a au moins un film en une composition polymère comprenant un complexe de bismuth en une quantité correspondant à 0,002 à 0,08 % en poids de la composition polymère, couvrant ladite surface.

19. Article selon la revendication 18, **caractérisé en ce que** ledit article a un revêtement multicouche de ladite composition polymère.

20. Article selon la revendication 18, **caractérisé en ce que** ledit article a un revêtement de ladite composition polymère et **en ce que** ladite composition polymère comprend en outre des nanoparticules, qui comprennent un complexe de bismuth.

21. Article selon la revendication 20, **caractérisé en ce que** ladite composition polymère comprend un composant polymère filmogène.

22. Article selon la revendication 21, **caractérisé en ce que** le polymère filmogène est un polymère de siloxane et/ou un oligomère de siloxane.

23. Article selon la revendication 21, **caractérisé en ce que** ladite composition polymère comprend en outre une séquence polymère hydrophobe ayant un ou plusieurs groupes terminaux réactifs.

24. Article selon la revendication 23, **caractérisé en ce que** le polymère filmogène est choisi dans le groupe constitué par les polyuréthanes, les polyoléfines, les élastomères, les polyéthylèneglycols, les polycarbonates, les polyéthersulphones, les polysulfones, les polyvinylpyrrolidones, et les chlorures de polyvinyle, et la séquence hydrophobe est choisie dans le groupe constitué par les polymères de siloxane, les oligomères de siloxane, les fluoropolymères, les polyéthylèneglycols, les copolymères de polyéthylèneglycol-polydiméthyl siloxane, les polyesters de silicone, et les copolymères triséquencés de polylactone-polysiloxane-polylactone.

25. Article selon la revendication 24, **caractérisé en ce que** le polymère filmogène est un polyuréthane et la séquence polymère hydrophobe est un copolymère triséquencé de polylactone-polysiloxane-polylactone contenant des groupes terminaux mono-, bi- ou multiréactifs.

26. Utilisation d'une composition polymère biocompatible selon la revendication 1, pour un dispositif médical destiné à être en contact avec le sang, le tissu, les plaies, les cellules dans des liquides de culture, les fluides biologiques, un fluide de dialyse ou les fluides thérapeutiques destinés à être éliminés ou perfusés de manière à améliorer la biocompatibilité et à empêcher la croissance bactérienne.

27. Utilisation d'une composition polymère biocompatible selon la revendication 1, sur des surfaces en contact avec le sang, le tissu, la peau, les couches épithéliales, les plaies, les cellules dans des liquides de culture, les fluides biologiques, les fluides de dialyse et/ou fluides thérapeutiques destinés à être éliminés ou à être perfusés.

28. Composition polymère biocompatible selon la revendication 9, **caractérisée en ce que** la libération de bismuth de ladite composition polymère dans une solution aqueuse est inférieure à 0,05 % de la quantité totale du bismuth contenu dans la composition polymère.

29. Article selon la revendication 18, dans lequel au moins un film est constitué par une composition polymère biocompatible selon la revendication 9, **caractérisé en ce que** la libération de bismuth dudit au moins un film dans la solution aqueuse est inférieure à 0,05 % de la quantité totale de bismuth contenue dans la composition polymère.
